# EUROPÄISCHE PATENTSCHRIFT

(11) **EP 1 021 416 B1**
(45) Veröffentlichungstag und Bekanntmachung des Hinweises auf die Patenterteilung: **19.05.2004**
(21) Anmeldenummer: 98967139.1
(22) Anmeldetag: 22.06.1998
(51) Int. Cl.: C07D 231/12

(54) **VERFAHREN ZUR HERSTELLUNG SUBSTITUIERTER PYRAZOLE**
METHOD FOR PRODUCING SUBSTITUTED PYRAZOLES
PROCEDE DE FABRICATION DE PYRAZOLES SUBSTITUES

(30) Priorität: 23.06.1997 DE 19726573
(43) Veröffentlichungstag der Anmeldung: 26.07.2000
(73) Patentinhaber: BASF AKTIENGESELLSCHAFT, 67056 Ludwigshafen (DE)
(72) Erfinder: Merkle, Hans Rupert, 67061 Ludwigshafen (DE); Fretschner, Erich, 69239 Neckarsteinach (DE)
(74) Vertreter: Kinzebach, Werner, Dr.
(86) Internationale Anmeldenummer: PCT/EP1998/003814
(87) Internationale Veröffentlichungsnummer: WO 1998/058914

(56) Entgegenhaltungen:
- EP-A- 0 402 722
- EP-A- 0 474 037
- WO-A-95/06036

## Beschreibung

Die vorliegende Erfindung betrifft ein Verfahren zur Herstellung von Pyrazolderivaten.

In "The Chemistry of Heterocyclic Compounds" Band 22, Kapitel 3 und 5, werden zahlreiche Synthesewege zum Pyrazol beschrieben, zum Beispiel die Kondensation von α,β-Dicarbonylverbindungen mit Hydrazinen, die Umsetzung von Ethinylcarbonylverbindung mit Hydrazinen und die Kondensation von Hydrazinoessigester mit 1,2-Diketonen.

Es ist ferner bekannt, 2-Pyrazolin mit Chlor, Alkalimetall- oder Erdalkalimetallhypochloriten (DE-A 30 35 395), mit Schwefel oder Selen (DE-A 30 29 160) oder mit wässrigem Wasserstoffperoxid (DE-A 34 15 385) zum Pyrazol zu dehydrieren. Weiterhin sind die thermische Gasphasendehydrierung von 2-Pyrazolin an Palladium- oder Platinkontakten (DE-A 32 09 148) sowie die Thermolyse von N-Sulfonyl-2-pyrazolin zum Pyrazol (DE-A 30 35 394) bekannt.

Außerdem ist die Dehydrierung von 2-Pyrazolinen in Schwefelsäure in Gegenwart von Iodverbindungen beschrieben. In der EP 0 474 037 wird das Pyrazolin in situ aus einem gegebenenfalls substituierten Hydrazin und But-2-endiol-(1,4), But-1-endiol-(3,4) oder Ethinylalkylcarbinol erzeugt. In der WO 95/06036 wird zunächst aus einem gegebenenfalls substituierten Hydrazin und einer α,β-ungesättigten Carbonylverbindung das Pyrazolin hergestellt und dann nach Mischen mit Schwefelsäure und dem Iodkatalysator dehydriert. In der EP 0 402 722 wird das Pyrazolin zuvor oder in situ aus einem gegebenenfalls substituierten Hydrazin und einem Glyzerin, einem Acrolein bzw. Vinylalkylketon oder einem β-Hydroxyethylalkylketon hergestellt.

Diese Verfahren sind jedoch technisch unbefriedigend, sei es, dass sie die Verwendung von sehr aggressiven Oxidationsmitteln oder teuren Katalysatoren erfordern, dass sie mit der Bildung giftiger Nebenprodukte, wie Schwefel- und Selenwasserstoff verbunden sind, dass die eingesetzten Verbindungen nur schwer zugänglich sind oder weil sie mehrere Verfahrensschritte umfassen.

Der Erfindung lag daher die Aufgabe zugrunde, ein Verfahren zur Herstellung von Pyrazolderivaten bereitzustellen, das technisch einfacher und wirtschaftlicher durchführbar ist.

Überraschend wurde nun gefunden, dass diese Aufgabe gelöst wird durch ein Verfahren zur Herstellung von Pyrazolderivaten der Formel I 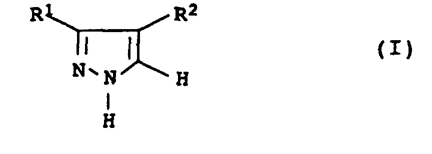 worin
- R¹ und R²: unabhängig voneinander für eine C₁-C₈-Alkyl-, C₃-C₈-Cycloalkyl-, C₆-C₁₄-Aryl-, Phenyl-C₁-C₄-alkylgruppe oder Tolyl stehen, worin die C₁-C₈-Alkyl-, C₃-C₈-Cycloalkyl-, C₆-C₁₄-Aryl- oder Phenyl-C₁-C₄-alkylgruppe unsubstituiert oder durch ein oder mehrere Halogenatome, Nitro-, Sulfo- oder Sulfonsäuregruppen substituiert sein kann, und
- R¹: zusätzlich auch Wasserstoff bedeuten kann,
dadurch gekennzeichnet, dass man eine Carbonylverbindung der Formel II 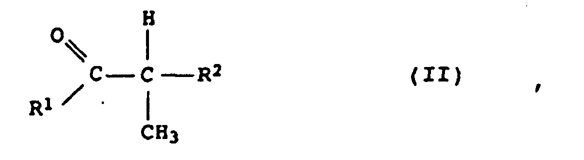 in der R¹ und R² die oben angegebenen Bedeutungen besitzen, mit Hydrazin, Hydrazin-Hydrat oder einem Säureadditionssalz davon, in Gegenwart von 30 bis 100 gew.-%iger Schwefelsäure und 0,05 bis 5 mol-% Iod bzw. einer Iod oder Iodwasserstoff freisetzenden Verbindung, bezogen auf die Hydrazinverbindung, bei Temperaturen im Bereich von 80 bis 200 °C umsetzt, wobei man die Hydrazinverbindung und die Carbonylverbindung in einem molaren Verhältnis von 1:0,8 bis 1:1,5 einsetzt.

Für das erfindungsgemässe Verfahren sind carbonylverbindungen der allgemeinen Formel II geeignet, worin R¹ und R² unabhängig voneinander ausgewählt sind unter einem geradkettigen oder verzweigten C₁-C₈-Alkyl, insbesondere C₁-C₄-Alkyl, wie Methyl, Ethyl, Propyl, 1-Methylethyl, Butyl, 1-Methylpropyl, 2-Methylpropyl und 1,1-Dimethylethyl; C₃-C₈-Cycloalkyl, wie insbesondere Cyclopentyl, Cyclohexyl und Cycloheptyl; C₆-C₁₄-Aryl, wie insbesondere Phenyl; Aralkyl, wie insbesondere Phenyl-C₁-C₄-alkyl, wobei der Alkylrest wie oben definiert ist, z. B. Benzyl und 2-Phenylethyl; und entsprechende organische Reste, die durch ein oder mehrere Halogenatome, wie F, Cl, Br oder I, Nitro-, Sulfo- oder Sulfonsäuregruppen substituiert sind, wie insbesondere Chlorphenyl, Nitrophenyl oder Tolyl. R¹ kann zusätzlich für Wasserstoffstehen.

Insbesondere steht R² für Methyl. Besonders bevorzugt steht R² für Methyl. Bevorzugt werden weiterhin Verbindungen der allgemeinen Formel II worin R¹ für Wasserstoff, Methyl, Ethyl, n-Propyl, tert.-Butyl, Phenyl, o-, m- oder p-Tolyl, o-, m- oder p-Chlorphenyl, o-, m- oder p-Nitrophenyl, o-, m- oder p-Sulfophenyl oder o-, m- oder p-Sulfonylphenyl stehen.

Insbesondere sind folgende Carbonylverbindungen geeignet: Isobutyraldehyd, Methylisopropylketon (2-Methylbutanon-3), Ethylisopropylketon (2-Methylpentanon-3), n-Propylisopropylketon (2-Methylhexanon-3), Isopropyl-t-butylketon, Phenylisopropylketon, Tolylisopropylketon, Chlorphenylisopropylketon, Nitrophenylisopropylketon, Sulfophenylisopropylketon und Sulfonylphenylisopropylketon.

Als zweite Reaktionskomponente wird Hydrazin verwendet. Es kann sowohl die freie Hydrazinbase als auch deren Hydrate oder Additionssalze mit Mineralsäuren, wie z. B. die Hydrazinsalze von Schwefelsäure, Salzsäure oder Phosphorsäure, eingesetzt werden. Da bei Einsatz von Salzen, die sich im Reaktionsmedium nicht lösen, Ausbeuteverluste auftreten können, wird bevorzugt das Hydrat oder die freie Base verwendet.

Schwefelsäure wird im erfindungsgemäßen Verfahren als Verdünnungs-, Kondensations- und als Oxidationsmittel verwendet. Ihre Konzentration liegt vorzugsweise im Bereich von 30 bis 100 Gew.-%, insbesondere im Bereich von 45 bis 90 Gew.-%.

Gegebenenfalls können inerte, organische Lösungsmittel, wie chlorierte Kohlenwasserstoffe, z. B. Dichlorethan, zusätzlich als Verdünnungsmittel eingesetzt werden.

Als Katalysator können neben elementarem Iod auch Iodverbindungen, wie Iodwasserstoff, Alkalimetall- und Erdalkalimetalliodide, wie Lithiumiodid, Natriumiodid, Kaliumiodid, Cäsiumiodid, Magnesiumiodid und Calciumiodid sowie sonstige Metalliodide zur Anwendung kommen. Es können auch andere, anorganische Iodverbindungen, wie Alkalimetall- oder Erdalkalimetallhypoiodite, -iodite, -iodate und -periodate, oder organische Iodverbindungen, wie Alkyliodide, z. B. Methyliodid, verwendet werden. Iod bzw. die Iodverbindung wird bei dieser Umsetzung im Allgemeinen in Mengen von 0,05 bis 5 mol-% pro Mol Hydrazin eingesetzt.

Die Umsetzung kann bei Verwendung von Hydrazinhydrat und Methylisopropylketon sowie Iodwasserstoff als Katalysator durch folgende Reaktionsgleichungen wiedergegeben werden:

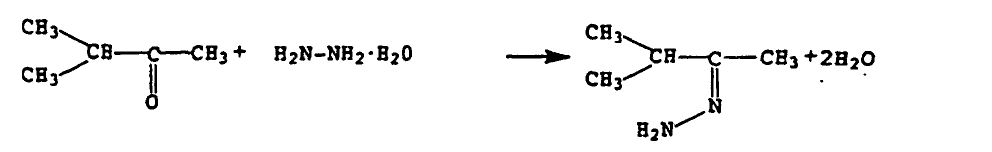

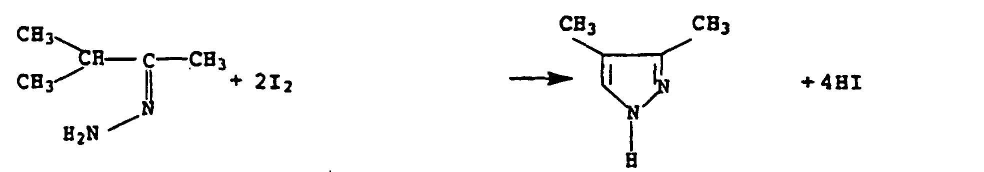

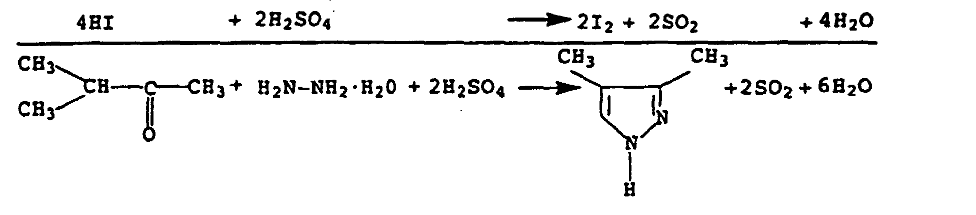

Bei Verwendung von Isobutyraldehyd als Carbonylverbindung, Hydrazinhydrat und Iodwasserstoff als Katalysator kann das Verfahren durch folgende Reaktionsgleichungen dargestellt werden:

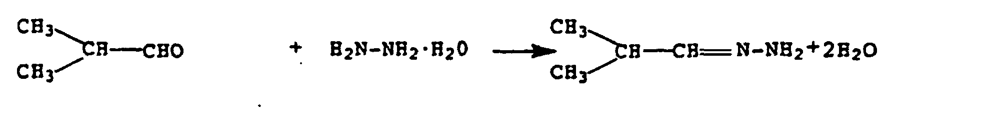

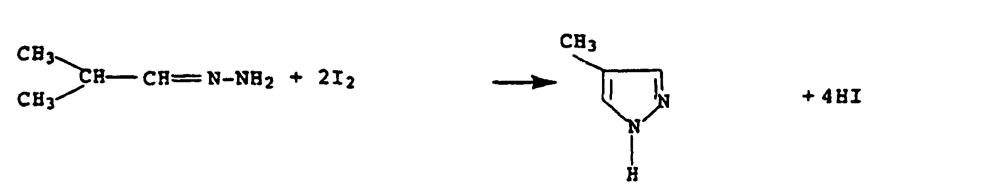

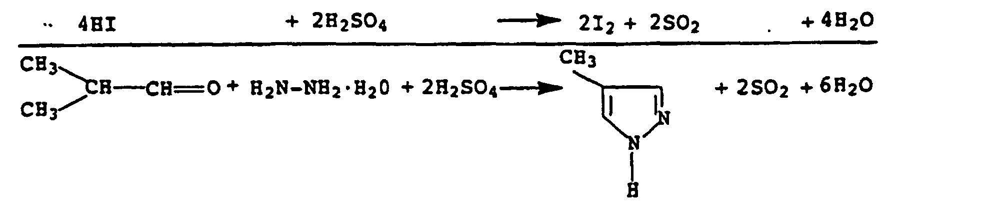

Die Umsetzung wird zweckmäßig so durchgeführt, dass man 1 Mol der Hydrazinverbindung mit 0,8 bis 1,5 Mol der Carbonylverbindung der Formel II in Schwefelsäure in Anwesenheit katalytischer Mengen einer Iodverbindung umsetzt, wobei das in der Reaktionsmischung vorhandene und zusätzlich gebildete Wasser teilweise entfernt werden kann. vorzugsweise erfolgt diese Abtrennung durch Destillation, beispielsweise unter Normaldruck, d. h. bei etwa 1 atm. Die Reaktionstemperaturen liegen im Bereich von 80 bis 200 °C und insbesondere von 110 bis 170 °C. Üblicherweise wird die Umsetzung bei Normaldruck durchgeführt. Es ist auch möglich, die Umsetzung bei erhöhtem Druck oder bei entsprechend erhöhter Temperatur in geringer konzentrierter Schwefelsäure bzw. bei verringertem Druck oder bei entsprechend niedriger Temperatur in höher konzentrierter Schwefelsäure durchzuführen.

Die Reaktion kann so durchgeführt werden, dass man alle Reaktionspartner in einem Reaktionsgefäß vorlegt und auf Reaktionstemperatur bringt, dass man die Reaktanden einem bei Reaktionstemperatur befindlichen Reaktionsgefäß als Mischung oder getrennt voneinander zuführt, oder dass man einen Teil der Reaktanden bei Reaktionstemperatur vorlegt und die verbleibende Menge im Verlauf der Reaktion zuführt. Es kann auch die Schwefelsäure selbst oder Hydrazin-Schwefelsäure im Reaktionsgefäß vorgelegt werden.

Die Reaktionstemperatur wird vorzugsweise unter Abdestillieren von Wasser erreicht. Das Einsetzen der Pyrazolbildung erkennt man an der Schwefeldioxidentwicklung. Das Schwefeldioxid ergibt bei Absorption mit Natronlauge molare Mengen Natriumhydrogensulfitlösung von hoher Reinheit. Das abdestillierte Wasser enthält den größten Teil des eingesetzten Iodids als Iodwasserstoff, der wieder zurückgeführt werden kann.

Die Aufarbeitung der Reaktionsmischung zur Isolierung des Pyrazols erfolgt durch übliche Verfahren. Bevorzugt neutralisiert man zur Aufarbeitung die dunkelbraune Reaktionsmischung, z. B. mit Natronlauge, Ammoniak oder anderen anorganischen Basen. Zur Isolierung des Pyrazols wird die neutralisierte Reaktionsmischung z. B. mehrfach mit einem Lösungsmittel extrahiert. Als Lösungsmittel eignen sich beispielsweise Isobutanol, chlorierte Kohlenwasserstoffe oder Tetrahydrofuran. Nach Trocknung der Extraktionslösung und Eindampfung zur Trockne erhält man die entsprechenden Pyrazole in 80 bis 90%iger Reinheit. Diese Rohprodukte können zur Verbesserung der Reinheit destilliert oder umkristallisiert werden. Die neutralisierte Reaktionsmischung kann auch destillativ aufgearbeitet werden, wobei Wasser und reines Pyrazol abdestilliert werden und durch organische Nebenprodukte verunreinigtes Natriumsulfat (oder Ammoniumsulfat) als Destillationsrückstand zurückbleibt. Bei Neutralisation mit Ammoniak wird im Destillationssumpf verunreinigtes Ammoniumsulfat erhalten, das oxidativ in Stickstoff und Schwefeldioxid gespalten werden kann. Letzteres kann wieder über SO₃ zu Schwefelsäure umgesetzt werden.

Das Verfahren kann kontinuierlich oder diskontinuierlich, drucklos, unter Druck oder schwachem Unterdruck durchgeführt werden.

Die nach dem erfindungsgemäßen Verfahren herstellbaren Pyrazol-Verbindungen der Formel I sind Ausgangsstoffe für organische Synthesen, zum Beispiel von pharmazeutischen Produkten und Pflanzenschutzmitteln. Besonders bevorzugt stellt man mit dem erfindungsgemäßen Verfahren folgende Verbindungen her: 4-Methylpyrazol, 3,4-Dimethylpyrazol, 3-Ethyl-4-methylpyrazol, 3-n-Propyl-4-methyl-pyrazol, 3-t-Butyl-4-methylpyrazol, 3-Phenyl-4-methylpyrazol, 3-Tolyl-4-methylpyrazol, 3-Chlorphenyl-4-methylpyrazol und 3-Nitrophenyl-4-methylpyrazol

Die folgenden Beispiele dienen der Veranschaulichung des erfindungsgemäßen Verfahrens.

### Beispiele

### Beispiel 1: Herstellung von 3,4-Dimethylpyrazol (Verbindung der Formel I in der R¹ für Methyl, R² für Methyl steht)

In einer Suspension aus 560 g (4,0 mol) 70%iger Schwefelsäure, 62,5 g (1,0 mol) 80%igem Hydrazinhydrat und 1 g (6,67 mmol) Natriumiodid werden, beginnend bei 120 °C innerhalb von 3 Stunden 111,8 g (1,3 mol) 3-Methyl-2-butanon zugetropft. Die Reaktionstemperatur fällt während des Zulaufs auf 110 °C ab. Nach Zulaufende wird die Temperatur durch Abdestillieren von 210 ml Wasser innerhalb von 2 Stunden auf 130 °C gebracht und 30 Minuten bei dieser Temperatur nachgerührt. Nach Zugabe der abdestillierten 210 ml Wasser bei 100 °C wird abgekühlt und mit 655 g (4,1 mol) 25%iger Natronlauge auf pH 9 gestellt. Nach der Extraktion mit iso-Butanol wird die organische Phase eingeengt und anschließend im Vakuum destilliert.

Man erhält 79,9 g 3,4-Dimethylpyrazol mit einem Gehalt von 99,2 %. Das entspricht einer Ausbeute von 82,6 % d. Th.; Sdp.: 90 °C (bei 5 mbar). Die Identifikation erfolgte durch die physikalisch chemischen Daten.

### Beispiel 2: Herstellung von 3-Phenyl-4-methylpyrazol (Verbindung der Formel I in der R¹ für Phenyl, R² für Methyl steht)

In einer Suspension aus 490 g (3,0 mol) 60%iger Schwefelsäure; 31,25 g (0,5 mol) 80%igem Hydrazinhydrat und 0,5 g (3,33 mmol) Natriumiodid werden bei 125 °C innerhalb von 2 Stunden 74,8 g (0,505 mol) Isopropyl-phenylketon zugetropft. Nach einer Stunde Nachrühren bei 125 °C wird durch Abdestillieren von 155 ml Wasser die Temperatur auf 140 °C gebracht. Nach dem Abkühlen wird die Reaktionsmischung mit 640 g (4,0 mol) Natronlauge auf pH 7,5 gestellt. Nach Filtration und Trocknung wurde der Filterrückstand aus Ethanol umkristallisiert.

Man erhält 69,2 g hellbraune Kristalle mit Smp.: 115 °C und einem Gehalt von 97 % (HPLC), was einer Ausbeute von 85 % d. Th. entspricht. Die Identifikation erfolgt durch die physikalisch chemischen Daten.

### Beispiel 3: Herstellung von 4-Methylpyrazol (Verbindung der Formel I in der R¹ für H, R² für Methyl steht)

Zu einer Suspension aus 560 g (4,0 mol) 70%iger Schwefelsäure und 62,5 g (1,0 mol) 80%igem Hydrazinhydrat werden 1,0 g (6,67 mmol) Natriumiodid gegeben und bei 125 °C innerhalb von 2 Stunden 86,4 g (1,2 mol) Isobutyraldehyd mit einer Dosierpumpe unter die Suspensionsoberfläche gepumpt. Während und bis zu 100 Minuten nach der Zufuhr des Isobutyraldehyds wurden insgesamt 175 g Wasser abdestilliert, wobei die Temperatur der Reaktionsmischung gegen Ende auf 135 °C ansteigt. Die Reaktionslösung wird nach dem Abkühlen mit 820 g (5,125 mol) 25%iger Natronlauge auf pH 8,6 gestellt und mit Isobutanol extrahiert. Die vereinigten Extrakte werden am Rotationsverdampfer auf 82 g eingeengt und anschließend destilliert.

Der Hauptlauf (Sdp. 82 °C bei 7 mbar; 49 g) besteht zu 82 % 4-Methylpyrazol, welches durch Vergleich mit authentischem Material identifiziert wurde. Ausbeute: 49 % d. Th.

### Beispiel 4: Herstellung von 3-Ethyl-4-methylpyrazol (Verbindung der Formel I in der R¹ für Ethyl, R² für Methyl steht)

Zu einer Suspension aus 280 g (2,0 mol) 70%iger Schwefelsäure, 12,5 g (0,25 mol) 100%igem Hydrazinhydrat und 0,5 g (3,33 mmol) Natriumiodid werden bei 125 °C 27,5 g (0,275 mol) 2-Methyl-3-pentanon zugetropft. Nach Zulaufende wird die Temperatur innerhalb von 1 Stunde auf 110 °C gebracht und 6 Stunden bei dieser Temperatur nachgerührt. Das Reaktionsgemisch wird abgekühlt und mit 480 g (3,0 mol) 25%-iger Natronlauge auf pH 9 eingestellt. Nach der Extraktion mit iso-Butanol wird die organische Phase eingeengt und anschließend im Vakuum destilliert.

Man erhält 18,5 g 3-Ethyl-4-methylpyrazol mit Sdp.: 90 °C bei 5 mbar und einem Gehalt von 95 % (HPLC), was einer Ausbeute von 63,9 % d. Th. entspricht. Die Identifikation erfolgt durch Vergleich der physikalisch chemischen Daten mit einer authentischen Probe.

## Patentansprüche

1. Verfahren zur Herstellung von Pyrazolderivaten der Formel I 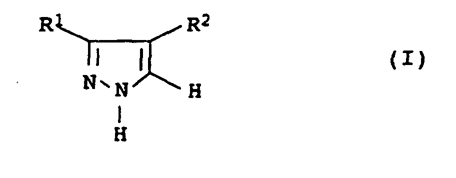 worin
R¹ und R² unabhängig voneinander für eine C₁-C₈-Alkyl-, C₃-C₈-Cycloalkyl-, C₆-C₁₄-Aryl-, Phenyl-C₁-C₄-alkylgruppe oder Tolyl stehen, worin die C₁-C₈-Alkyl-, C₃-C₈-Cycloalkyl-, C₆-C₁₄-Aryl- oder Phenyl-C₁-C₄-alkylgruppe unsubstituiert oder durch ein oder mehrere Halogenatome, Nitro-, Sulfo- oder Sulfonsäuregruppen substituiert sein kann, und
R¹ zusätzlich auch Wasserstoff bedeuten kann,
**dadurch gekennzeichnet, dass** man eine Carbonylverbindung der Formel II 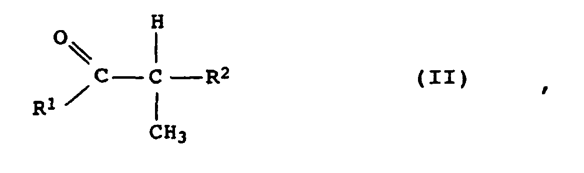 in der R¹ und R² die oben angegebenen Bedeutungen besitzen, mit Hydrazin, Hydrazin-Hydrat oder einem Säureadditionssalz davon, in Gegenwart von 30 bis 100 gew.-%iger Schwefelsäure und 0,05 bis 5 mol-% Iod bzw. einer Iod oder Iodwasserstoff freisetzenden Verbindung, bezogen auf die Hydrazinverbindung, bei Temperaturen im Bereich von 80 bis 200 °C umsetzt, wobei man die Hydrazinverbindung und die Carbonylverbindung in einem molaren Verhältnis von 1:0,8 bis 1:1,5 einsetzt.

2. Verfahren nach Anspruch 1, **dadurch gekennzeichnet, dass** man 45 bis 90 gew.-%ige, Schwefelsäure verwendet.

3. Verfahren nach einem der Ansprüche 1 bis 2, **dadurch gekennzeichnet, dass** man die Umsetzung bei Temperaturen im Bereich von 110 bis 170 °C durchführt.

4. Verfahren nach einem der Ansprüche 1 bis 3, **dadurch gekennzeichnet, dass** man die Umsetzung bei Normaldruck durchführt.

5. Verfahren nach einem der Ansprüche 1 bis 4, **dadurch gekennzeichnet, dass** man bei der Umsetzung anfallendes Wasser aus dem Reaktionsgemisch entfernt.

6. Verfahren nach einem der Ansprüche 1 bis 5, **dadurch gekennzeichnet, dass** man eine Carbonylverbindung der Formel II einsetzt, worin R¹ die oben angegebenen Bedeutungen besitzt und R² für Methyl steht.

7. Verfahren nach Anspruch 6, **dadurch gekennzeichnet, dass** man eine Verbindung erhält, worin R¹ für ein Wasserstoffatom, Methyl, Ethyl, n-Propyl, t-Butyl, Phenyl, Tolyl, Chlorphenyl, Sulfonylphenyl, Sulfophenyl oder Nitrophenyl steht und R² für Methyl stehen.

## Claims

1. A process for preparing pyrazole derivatives of the formula I 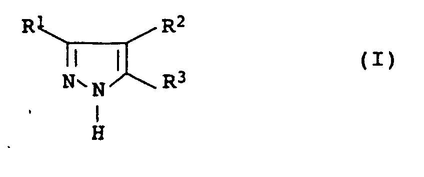 where R¹ and R² are, independently of one another, a C₁-C₈ alkyl, C₃-C₈ cycloalkyl, C₆-C₁₄ aryl, phenyl-C₁-C₄ alkyl group or tolyl, in which the C₁-C₈ alkyl, C₃-C₈ cycloalkyl, C₆-C₁₄ aryl or phenyl-C₁-C₄ alkyl group may be unsubstituted or substituted by one or more halogen atoms, nitro, sulfo or sulfonic acid groups and
R₁ may additionally be hydrogen,
which comprises reacting
a carbonyl compound of the formula II 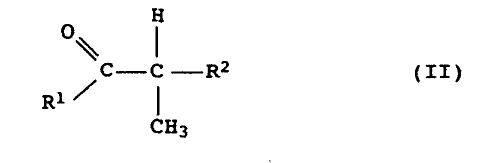 where R¹ and R² have the abovementioned meanings, with hydrazine, hydrazine hydrate or an acid addition salt thereof, in the presence of from 30% to 100% by weight sulfuric acid and 0.05 to 5 mol% iodine or of a compound which liberates iodine or hydrogen iodide based on the hydrazine compound at temperatures of from 80 to 200 °C, the hydrazine compound and the carbonyl compound being employed in a molar ratio of from 1:0.8 to 1:1.5.

2. A process as claimed in claim 1, wherein the sulfuric acid used is from 45 to 90% by weight.

3. A process as claimed in either of claims 1 to 2, wherein the reaction is carried out at from 110 to 170°C.

4. A process as claimed in any of claims 1 to 3, wherein the reaction is carried out under atmospheric pressure.

5. A process as claimed in any of claims 1 to 4, wherein water produced in the reaction is removed from the reaction mixture.

6. A process as claimed in any of claims 1 to 5, wherein a carbonyl compound of the formula II where R¹ has the stated meanings and R² is methyl is employed.

7. A process as claimed in claim 6, wherein a compound where R¹ is a hydrogen atom, methyl, ethyl, n-propyl, t-butyl, phenyl, tolyl, chlorophenyl, sulfophenyl or nitrophenyl and R² is methyl is obtained.

## Revendications

1. Procédé de préparation de dérivés de pyrazole de formule I 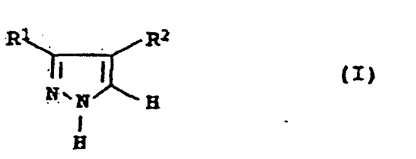 dans laquelle
R¹ et R² représentent indépendamment l'un de l'autre un groupe alkyle en C₁ à C₈, cycloalkyle en C₃ à C₈, aryle en C₆ à C₁₄, phényl-alkyle en C₁ à C₄ ou tolyle, où le groupe alkyle en C₁ à C₈, cycloalkyle en C₃ à C₈, aryle en C₆ à C₁₄ ou phényl-alkyle en C₁ à C₄ peut être non substitué ou substitué par un ou plusieurs atomes d'halogènes, groupes acides nitro-, sulfo- ou sulfonique,
et
R¹ peut en outre également représenter un hydrogène,
**caractérisé en ce qu'**on fait réagir un composé carbonyle de formule II 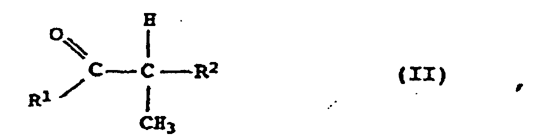 dans laquelle R¹ et R² ont les significations indiquées ci-dessus, avec de l'hydrazine, de l'hydrazine hydratée ou un de ses sels d'addition d'acides, en présepce d'acide sulfurique à 30 à 100 % en poids et de 0,05 à 5% molaire d'iode ou respectivement d'un composé libérant de l'iode ou de l'acide iodhydrique, par rapport au composé d'hydrazine, à des températures allant de 80 à 200 °C, opération dans laquelle on utilise le composé d'hydrazine et le composé carbonyle dans un rapport molaire de 1 :0,8 à 1 :1,5.

2. Procédé selon la revendication 1, **caractérisé en ce qu'**on utilise de l'acide sulfurique à 45 à 90% en poids.

3. Procédé selon l'une des revendications 1 à 2, **caractérisé en ce qu'**on conduit la réaction à des températures allant de 110 à 170 °C.

4. Procédé selon l'une des revendications 1 à 3, **caractérisé en ce qu'**on conduit la réaction à pression normale.

5. Procédé selon l'une des revendications 1 à 4, **caractérisé en ce que** dans la réaction on retire l'eau qui apparaît à partir du mélange réactionnel.

6. Procédé selon l'une des revendications 1 à 5, **caractérisé en ce qu'**on utilise un composé carbonyle de formule II dans laquelle R1 possède les significations indiquées ci-dessus et R2 représente un méthyle.

7. Procédé selon la revendication 6, **caractérisé en ce qu'**on obtient un composé dans lequel R1 représente un atome d'hydrogène, un méthyle, un éthyle, un n-propyle, un t-butyle, un phényle, un tolyle, un chlorophényle, un sulfonylphényle, un sulfophényle ou un nitrophényle et R2 représente un méthyle.
